Europäisches Patentamt

European Patent Office

Office européen des brevets

(19)

(11) Publication number: **0 232 600 B1**

## EUROPEAN PATENT SPECIFICATION

(12)

(45) Date of publication of patent specification: **24.04.91**   (51) Int. Cl.<sup>5</sup>: **A61M 1/00**

(21) Application number: **86309257.3**

(22) Date of filing: **27.11.86**

(54) Apparatus for draining fluid from a body cavity.

(30) Priority: **30.11.85 GB 8529524**

(43) Date of publication of application:
**19.08.87 Bulletin 87/34**

(45) Publication of the grant of the patent:
**24.04.91 Bulletin 91/17**

(84) Designated Contracting States:
**AT BE CH DE ES FR IT LI LU NL SE**

(56) References cited:
| EP-A- 0 113 520 | WO-A-85/04795 |
| GB-A- 1 394 632 | GB-A- 1 434 507 |
| GB-A- 1 491 186 | GB-A- 2 140 301 |
| US-A- 3 957 052 | |

(73) Proprietor: **SHERWOOD MEDICAL COMPANY**
**1831 Olive Street**
**St. Louis, MO 63103(US)**

(72) Inventor: **Katsaros, Georges**
**4 R. Mathieu Bodson**
**4500 Jupille(BE)**
Inventor: **Macors, Paul**
**132 Rue Naniot**
**4000 Liège(BE)**

(74) Representative: **Porter, Graham Ronald et al**
**C/O John Wyeth & Brother Limited Hunter-**
**combe Lane South Taplow**
**Maidenhead Berkshire, SL6 0PH(GB)**

Rank Xerox (UK) Business Services

## Description

This invention relates to apparatus for draining fluid from a body cavity and in particular to a needle assembly for pleural cavity drainage or abdominal paracentesis.

Certain medical conditions may result in an accumulation of unwanted pathological fluid in a body cavity. For example, the medical condition known as pleurisy is characterised by an accumulation of fluid in the pleural cavity which seriously impairs both inhalation and expiration and can ultimately result in death. Dangerous accumulation of fluid may also occur as a result of a surgical incision or a bullet or stab wound.

Suction or gravity drain apparatus is known for draining excess fluid from a body cavity via an implanted catheter. Typical surgical drainage apparatus is shown, for example, in U.S. Patent No. 3,363,626.

The fluid drainage path between the body cavity and drainage apparatus may be a simple tube connected at one end to the drianage apparatus and inserted at the other end through a surgical incision in the body cavity wall. Such an arrangement has a number of disadvantages particularly when draining the pleural cavity.

Firstly the patient's breathing will be seriously impaired as soon as the chest wall is breached, at a time when the patient may already be having breathing difficulties. Secondly the surgeon must be careful not to cut too deeply as he may damage lung tissue. Thirdly the insertion process must, as far as possible, be carried out aseptically to avoid infection, and the drainage apparatus itself must not allow the patient to become infected by reverse passage of bacteria. Fourthly the drainage catheter must not be inserted too deeply otherwise damage to lung tissue or occlusion of the open catheter end by lung tissue may result, and finally the drainage catheter must be secured to the patient's body, and the chest wall incision sealed to the wall of the catheter to allow the patient to breath unaided.

International Patent application WO 85/04795 discloses a thoracentesis device which purports to overcome some of the above mentioned problems. That device includes a needle assembly comprising several components and a valve or tap to control flow of fluid from the patient. Such an assembly has the disadvantage that infection may enter the drainage tract through a joint or through the tap; the tap might also be inadvertently turned the wrong way to expose the drainage path to atmosphere. Furthermore the user may dismantle and reassembly the apparatus prior to use so introducing infection, or incorrectly reassemble the apparatus which may result in infection and/or malfunction as will be hereinafter explained.

Another device is shown in United Kingdom Patent Application 2,140,301 and includes a clamp to fix the assembly to the patient's skin at the desired depth of insertion. This apparatus includes a retractable needle.

EPA-113520 discloses a nephroscopy cannula for providing a percutaneous access tract to the interior of the kidney, the cannula including a central passageway of size sufficient to accommodate at least one of various instruments, the cannula having a side port. The cannula has a cylindrical portion including an insertion portion adjacent one end which is sized for extending from the renal pelvis of the kidney of the body to the exterior of the body, the cylindrical portion including a non-insertion portion having an outer diameter substantially greater than the outer diameter of the insertion portion and means for temporarily securing the insertion portion and means for selectively closing the other end of the cannula e.g. a septum. The temporary securing means which is illustrated is a retention disc and tie down which is used to secure the inner portion of the disc to the cylindrical portion of the cannula. The retention disc is also secured to the skin of the patients body by sutures.

This cannula apparatus is unsuitable for draining a pleural cavity as the interior of the cannula is either continuously exposed to the environment or exposed to the environment each time the septum is removed from the proximal end of the cannula. The patients tissues would be exposed to the atmosphere for an unacceptably long period and the patients lung would collapse.

GB-A-1 394 632 concerns a surgical instrument comprising a cannula and a guard which is mounted on the cannula, is movable along the length of the cannula, and is adapted to be locked to the cannula, the guard consisting of male and female elements, the male element having a cylindrical spigot and the female element having a cylindrical cavity for receiving the spigot, both elements having through-going cylindrical channels accomodating the cannula and eccentric to the axes of the spigot and the cavity respectively, and rotation of either element with respect to the other, from a position in which the channels are aligned, being effective to cause frictional engagement between the outer surface of the spigot and the inner surface of the cavity and simultaneous locking of the guard to the cannula.

The present invention provides simplified and improved apparatus which overcomes the aforementioned problems whilst providing certain additional and improved features including a sealed drainage path which obviates the chance of infection entering the patient's body through the drainage apparatus.

According to the invention there is provided apparatus for draining the pleural cavity and comprising a housing defining an internal chamber in fluid communication with a cannula extending from said housing, an outlet port extending from said housing and an access port in axial alignment with said cannula and closed by a self sealing elastomeric plug,a removable needle passing through said plug into said cannula, a flange axially movable of the cannula and having a distal face characterised in that the cannula is integral with the housing, in that the internal chamber of the housing is in constant fluid communication with the outlet port and in that the apparatus includes a collet engageable with a recess on the proximal face of said flange to lock the flange against axial movement. The cannula is moulded as a single unit with the housing thus ensuring that infection cannot enter the apparatus via a joint or a tap. The outlet port may likewise be separately made and permanently joined to the housing.

In one embodiment the housing is transparent to allow the user to observe fluid passing therethrough from the pleural cavity. The presence of fluid in the housing is an indication that the cannula is correctly inserted.

A further advantage of integral construction is that the assembly is not affected by changes in internal pressure. A patient suffering from pleurisy can show a tendency to gasp suddenly or cough violently. Such involuntary chest movements can result in abnormally high and low pressures in the drainage apparatus especially where anti-reflux valves and the like are incorporated in the fluid collection vessel.

A drainage assembly formed of separable components or having moving parts may be adversely affected by such sudden changes in pressure and in particular may permit bacteria to enter the system in conditions of high vacuum through joints which otherwise provide an adequate seal.

Malfunction of a drainage assembly, such as an air leak through a disconnectable joint, can also have life threatening consequences for the patient. An air leak may result from a disconnectable joint not being adequately tight or a joint being loosened by repeated coughing or patient movement. The present invention obviates such possibilities.

The removable needle eliminates any danger of damage or puncture to lung or other cavity tissue from the needle point and overcomes the problem inherent in a retractable needle assembly, that the needle may inadvertently be returned to a position in which it protrudes from the cannula. A removable needle also helps to minimise the extent to which the assembly protrudes from the patient's skin which is generally more convenient and comfortable.

The self-sealing plug ensures sterility of the cannula/outlet port tract after the needle has been removed. Prior to removal the needle may be used to introduce medication or antiseptic into the pleural cavity in a conventional manner since the proximal end of the needle is free for attachment to other medical devices e.g. a syringe as will be hereinafter described. After the needle is removed the access port permits the introduction of medication or antiseptic directly into the body cavity by syringe and hypodermic needle through the self-sealing plug. This is an improvement over prior art devices having an access port downstream of the needle assembly. In these devices medication must not only be pumped in against the flow of draining fluid but the entire fluid tract must be filled with medication thus perhaps giving the patient an imprecise dose.

The assembly may be provided with an integral drainage tube and collection bag thus providing a sealed unit ready for immediate use. Such a unit can be taken in sterilized condition from a factory sealed pack in the operating theatre and placed in a patient for immediate use.

It is of crucial importance that the cannula tract is maintained in a sterile condition without any chance of air borne infection entering the body through the cannula. The device according to the invention has no moving parts or other openings through which infection could pass. A closure tap or valve may be necessary in the outlet tube to shut off flow to the collection chamber but this is preferably a finger operated roller clamp of known type which closes the drain tube by squeezing the tube wall. In one embodiment of the invention the fluid tract from cannula to collection container is completely sealed.

The self-sealing plug may be secured in the access port by any convenient means, so long as variations in internal pressure are insufficient to cause the plug to be dislodged and/or provide a leakage path. Preferably the plug has a peripheral flange to limit insertion into the access port. Such a flange fulfills the dual purpose of placing the plug in the desired position in the housing and resisting the tendency of internal vacuum to dislodge the plug. The flange has the advantage that internal vacuum tends to draw the flange against the abutting wall of the access port so further reducing the chance of an air leak.

Fluid passing through the drainage assembly is often infected and may contain a high proportion of blood. It is desirable to minimise dead space in the flow path to ensure that infected fluid is passed directly to the collection chamber. If infection is retained in the assembly by, for example, clotted blood, there is a dnager of infection re-entering the cavity and countering the effect of antibiotics administered to the patient. Accordingly the depth of insertion of the plug is designed to minimise dead space within the housing between the outlet port and access port.

In the preferred emnodiment a cap is provided for the access port. The cap is attached to the housing by any convenient means and has a single opening in axial alignment with the cannula for passage of the hollow needle. The cap serves the dual purpose of retaining the plug against excessive positive pressure within the housing, such as when a patient coughs, and further reducing the possibility of infection entering the drainage apparatus.

A cover may be provided to close the cap opening when the needle is withdrawn.

In a preferred embodiment the outlet port is generally perpendicular to the axis of the cannula. In this way the protrusion of the assembly above the patient's skin is minimised and the necessary drainage tube more conveniently attached to the patient's skin without fear of kinking.

The apparatus also includes means to retain the cannula at a desired depth of insertion. In the preferred embodiment the flange has an adhesive distal face.

The collet arrangement grips the cannula around the entire periphery thereof so giving a secure fixing whilst exerting minimum crushing force on the cannula wall.

The collet may comprise a hub having a plurality of distally extending fingers for engagement with the recess of the flange. The recess may taper inwardly in a distal direction to urge the fingers of the collet against the cannula as the collet is pushed into the flange recess.

The distal face of the flange may be coated with any suitable medically acceptable adhesive which will adhere to the patient's skin. The adhesive layer is preferably covered with a protective film which is peeled off immediately prior to use.

In the preferred embodiment two one-way valves are provided in series in the drain tube to permit outflow only; a pump port is provided intermediate the valves for attachment to a syringe. Outward movement of the syringe plunger will draw fluid through the upstream valve into the syringe body and inward movement will force fluid from the syringe body through the downstream valve into the collection chamber. Such apparatus may be useful for single person operation where pumped drainage is required. The syringe may be left attached to the pump port when not in use and will not impede drainage. The syringe plunger may be left in an extended position to exert a slight suction on the body cavity and so enhance drainage.

Other features of the invention will be apparent from the following description of a preferred embodiment shown by way of example only with reference to the accompanying drawings in which:-

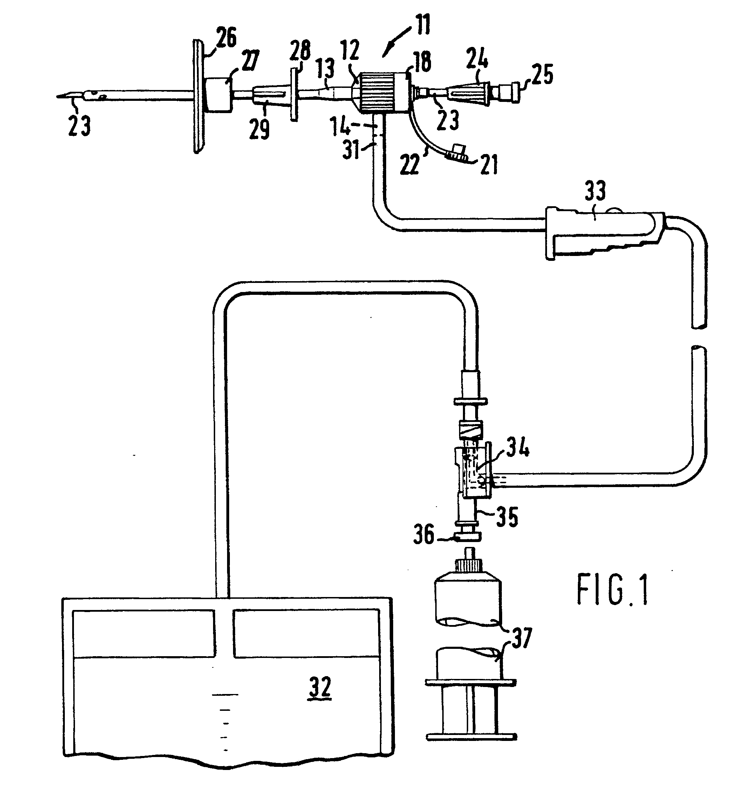

Figure 1 shows a drainage needle assembly and associated apparatus suitable for draining a body cavity of excess fluid; and

Figure 2 is an axial part-section through the components of the needle assembly of Figure 1 shown in exploded form.

With reference to the drawings there is shown a needle assembly 11 comprising a housing 12 having an integral cannula 13 extending therefrom and an outlet port 14. The outlet 14 is shown as a separate component and is fixed to the housing 12 by any suitable means which obviates an infection path. The cannula has a rounded end to avoid damage to lung tissue, and side eyes to allow fluid to pass into the cannula if the open end should be occluded, for example by lung tissue. The housing has an internal chamber 15 to place the cannula 13 and outlet port 14 in fluid communication, and has a proximal access port 16 closed by a resilient plug 17. The plug 17 includes a peripheral flange 20 to limit insertion into the access port 16. The flange 20 fulfills the dual purpose of resisting abnormally low pressures which can exist in the chest cavity and controlling the depth of insertion to minimise dead space in the housing 12 whilst not occluding the outlet port 14. The plug 17 is of a grade of rubber which is self-sealing when a needle placed therethrough is removed, and may be retained in the access port by any convenient means, for example friction or adhesive.

An annular cap 18 locates on a shoulder of the housing 12 and is bonded thereto, for example by adhesive. The cap 18 retains the plug 17 in housing 12 and has an axial opening 19 which is closable by a cover 21 secured to the cap 18 by a flexible strap 22.

A hollow steel needle 23 passes through the opening 19, plug 17 and housing 12 to protrude from the cannula 13 by a small amount as shown. The plug 17 grips the needle to maintain both a predetermined axial relationship between the needle and cannula and asceptic conditions distally of the plug 17 as will become apparent.

The needle 23 has a conventional luer hub 24 which is closed by a filter 25; the hub 24 is preferably transparent. The hub 24 may include a key to register with a slot of the cap 18. This helps to locate the needle relative to the housing 12 which avoids needle rotation as the body cavity is pierced.

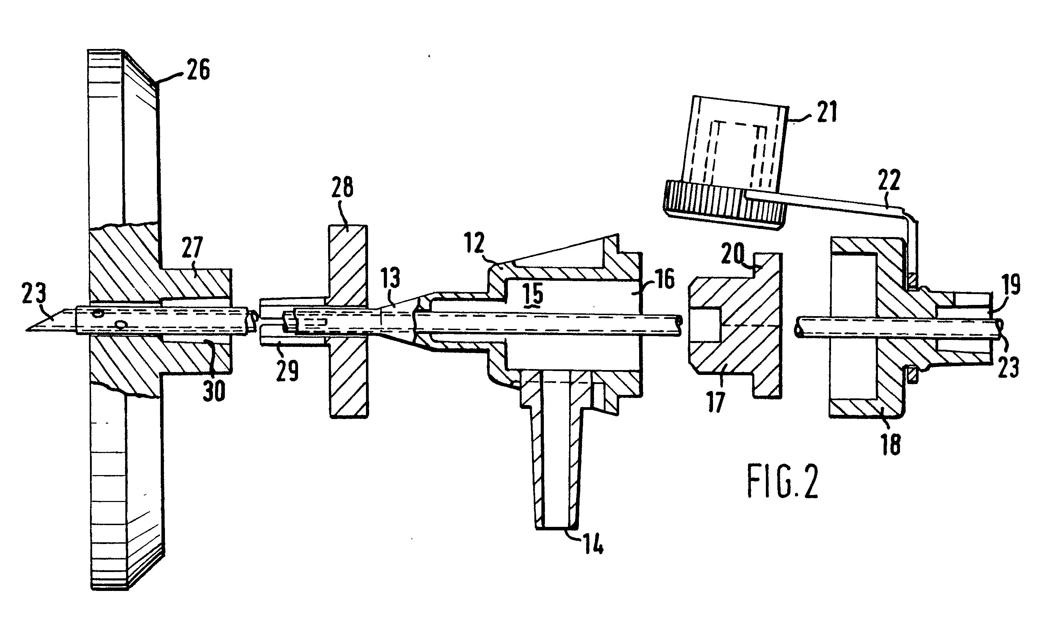

An annular flange 26 surrounds the cannula 13 and has a distal face coated with a skin compatible adhesive which may be, for example, a thin sheet of adhesive foam. The adhesive coating is normally covered with a protective layer which is peeled off prior to use. The flange 26 has an axially extending hub 27 adapted to receive an annular locking member or collet 28. The collet 28 has a four-section jaw 29 for insertion into a recess 30 of the hub 27 thereby to grip the cannula 13 and hold the flange in a desired position. The collet may have less or more jaw sections, a two section jaw will work effectively for example. With reference to Figure 2 the recess 30 is shown having an internal taper whose angle is chosen both to force the individual jaws of the collet 28 into contact with the cannula 13 and to retain the split jaw 29 in the recess 30 by frictional forces alone. The members of the split jaw are urged into frictional engagement with the cannula and exert a generally uniform radial force thereon. The dimensions of the recess 30, cannula 13 and collet 28 are chosen to ensure that adequate frictional clamping forces are generated without imposing an excessive crushing force on the cannula wall.

The outlet port 14 is connected by flexible medical tubing 31 to a collection container 32. A roller clamp 33 is provided on the tubing 31. The container may be a flexible bag of, for example, polythene.

In the preferred embodiment the drainage tube includes a valve assembly 34 comprising two one-way valves which permit outflow only; a pump port 35 intermediate the valves gives access to the fluid drainage path. The port 35 is closed by a removable plug 36. A syringe 37 may be attached to the port 35 to act as a pump as will be further described hereinafter.

Component parts of the apparatus may be made from any suitable medical grade of plastic or rubber. The apparatus is sterilized after manufacture and maintained in a sterile condition prior to use, for example by enclosure in an hermetically sealed bag.

In use, the surgeon cleans the skin area to be punctured and removes the apparatus from its sealed enclosure. The flange 26 may be adjusted and locked on the cannula to prevent accidental excessive penetration. The flange may also provide a visual indication of penetration depth in conjunction with markings provided on the cannula wall.

As the needle is inserted into the cavity to be drained, fluid passes through the needle to the transparent hub 24 and provides a visual indication to the surgeon that the desired depth of penetration has been reached. The filter 25 allows air to escape as fluid flows to the hub. In cases where fluid is unlikely to flow freely to the hub 24, the surgeon may remove the filter 25 and attach a conventional syringe to draw fluid from the body cavity.

When the surgeon is satisfied that the needle assembly is correctly inserted, the protective layer is stripped from the adhesive face of the flange 26 and the flange secured both to the patient's skin and, using the collet 28, to the cannula 13. The position of the flange may be varied by simply releasing the collet, repositioning the flange or cannula and re-fixing the collet.

The needle 23 may then be completely removed from the assembly and the cover 21 secured in place. The opening in the plug 17 closes as the needle is removed and seals the chamber 15. The distal face of the plug 17 closes before the needle is completely removed, thus obviating the chance that airborn infection may enter. The cover 21 provides an additional safety seal.

The surgeon then releases the roller clamp 32 to allow fluid to drain from the body cavity into the collection chamber either by gravity or under active suction. The transparent housing 12 confirms fluid flow.

The needle 23 may be used to obtain a sample of fluid from the body cavity or to introduce medication before the surgeon commences fluid drainage. In this procedure a conventional syringe is attached to the luer hub 24 to draw a sample through the needle for examination or analysis. After the needle 23 has been withdrawn from the assembly a surgeon may take a fluid sample at any time by opening the cover 21 and pushing a hypodermic needle of a conventional syringe through the plug 17 into the chamber 15. The sample is drawn into the syringe, and the syringe and needle removed after which the cover 21 is replaced. Medication may be introduced through the plug 17 directly into the body cavity in the same manner. The natural resiliency of the plug ensures that the aperture made by the needle is closed as soon as the needle is withdrawn. Each needle insertion will almost certainly pierce the plug at a fresh position so obviating the introduction of infection through a previously used site.

In some medical procedures it is necessary to aspirate fluid from the cavity and for this purpose a syringe may be attached to the pump port 35. Outward movement of the syringe plunger will draw fluid through the upstream one-way valve into the syringe body; inward movement of the plunger will force fluid through the downstream one-way valve into the fluid collection container. This arrangement provides a simple pump unit that can be operated by a single person when required. The syringe can remain attached to the pump port when not in use and will not impede gravity drainage. A further advantage is that the syringe plunger can be left in an extended position to exert a slight suction on the body cavity and so enhance drainage.

Clearly the dimensions of the individual components of the assembly are chosen to suit the patient and the cavity to be drained. Specifically the length of the cannula may be chosen to minimise the length upstanding from the patient's body after insertion. Other fittings or adapters may be provided on the needle 23 as required for specific surgical procedures.

The assembly is provided in a sterile condition without any openings or moving parts through which infecting organisms could enter the fluid duct. The sole opening is that through which the needle is withdrawn and which is immediately closed by the plug 17. At no time should any non-sterile surface of the needle pass to the interior of the housing 12.

The invention has been described with the double check valve assembly 34; the upstream valve ensures that infection cannot enter the drainage tract via the pump port 35. In any event normal surgical procedures would ensure that an attachment to the pump port will be sterile.

The double check valve assembly can be omitted and the apparatus provided with an integral collection bag; the bag would be sealed except for a filter in the wall thereof to permit air flow outwardly.

Where other drainage apparatus is used, for example a suction drainage unit, the downstream end of the drainage tube may be provided with a removable cap to facilitate connection to the inlet port of such apparatus.

Whilst the invention has been described in relation to pleural cavity drainage, the needle assembly is quite suitable for draining other cavities containing accumulated fluid.

## Claims

1. Apparatus for draining the pleural cavity and comprising a housing (12) defining an internal chamber (15) in fluid communication with a cannula (13) extending from said housing, an outlet port (14) extending from said housing and an access port (16) in axial alignment with said cannula and closed by a self sealing elastomeric plug (17), a removable needle (23) passing through said plug into said cannula, and a flange (26) axially movable of the cannula (13) and having a distal face, characterised in that the cannula (13) is integral with the housing (12), in that the internal chamber (15) of the housing is in constant fluid communication with the outlet port (14) and in that the apparatus includes a collet (28) engageable with a recess (30) on the proximal face of said flange to lock the flange (26) against axial movement.

2. Apparatus according to Claim 1, characterised in that a cap (18) is provided for said access port (16), the cap (18) being attached to said housing (12) and having an opening in axial alignment with the cannula (13) for passage of said needle (23).

3. Apparatus according to Claim 1 or Claim 2, characterised in that said plug (17) includes a peripheral flange (20) to limit insertion of the plug (17) into said access port (16).

4. Apparatus according to Claim 3, characterised in that the outlet port (14) is intermediate the cannula (13) and access port (16) and the distal face of said plug (17) is adjacent said outlet port (14).

5. Apparatus according to any preceding Claim characterised in that it further includes a drainage tube (31) sealed at one end to said outlet port (14), two one-way valves (34) being placed in series in said drainage tube (31) to permit outflow only and said tube (31) having a pump port (35) intermediate said valves to give access to the interior of the tube.

6. Apparatus according to Claim 5 characterised in that it further includes a syringe (37) attached to said pump port (35) such that the interior of the syringe is in fluid communication with the interior of said tube (31).

7. Apparatus according to Claim 5 or Claim 6, characterised in that the other end of said tube (31) is sealed to the inlet of a fluid receptacle.

8. Apparatus according to any one of the preceding Claims characterised in that said collet (28) comprises a hub slidable on said cannula (13) and having a plurality of distally extending fingers (29) for engagement in said recess of the flange (26).

9. Apparatus according to Claim 8, characterised in that said recess (30) tapers radially inwardly in a distal direction.

10. Apparatus according to any one of the preceding Claims, characterised in that the flange (26) has an adhesive distal face.

## Revendications

1. Appareil pour drainer la cavité pleurale comprenant un boîtier (12) définissant une chambre interne (15) en communication de fluide avec une canule (13) partant du boîtier, une lumière de sortie (14) partant du boîtier et une lumière d'accès (16) axialement en ligne avec la canule et fermée par un bouchon élastomère à autoétanchéité (17), un trocart amovible (23) traversant le bouchon et pénétrant dans la canule, et une collerette (26) axialement mobile par rapport à la canule (13) et comportant une face distale, caractérisé en ce que la canule (13) fait partie intégrante du boîtier (12), que la chambre interne (15) du boîtier est en communication de fluide constante avec la lumière de sortie (14) et que l'appareil comprend un mandrin (28) pouvant être engagé avec un évidement (30) prévu dans la face proximale de la collerette pour bloquer la collerette (26) de manière à l'empêcher de se déplacer axialement.

2. Appareil suivant la revendication 1, caractérisé en ce qu'un chapeau (18) est prévu pour la lumière d'accès (16), le chapeau (18) étant attaché au boîtier (12) et présentant une ouverture axialement en ligne avec la canule (13) pour le passage du trocart (23).

3. Appareil suivant la revendication 1 ou 2, caractérisé en ce que le bouchon (17) comprend une bride périphérique (20) destinée à limiter l'insertion du bouchon (17) dans la lumière d'accès (16).

4. Appareil suivant la revendication 3, caractérisé en ce que la lumière de sortie (14) est située entre la canule (13) et la lumière d'accès (16) et la face distale du bouchon (17) est adjacente à la lumière de sortie (14).

5. Appareil suivant l'une quelconque des revendications précédentes, caractérisé en ce qu'il comprend, en outre, un tube de drainage (31) scellé de manière étanche à une extrémité à la lumière de sortie (14), deux valves antiretour (34) étant placées en série dans le tube de drainage (31) pour ne permettre qu'un écoulement vers l'extérieur et le tube (31) comportant une lumière de pompage (35) entre les valves, qui donne accès à l'intérieur du tube.

6. Appareil suivant la revendication 5, caractérisé en ce qu'il comprend, en outre, une seringue (37) attachée à la lumière de pompage (35), de telle sorte que l'intérieur de la seringue soit en communication de fluide avec l'intérieur du tube (31).

7. Appareil suivant la revendication 5 ou 6, caractérisé en ce que l'autre extrémité du tube (31) est adaptée de manière étanche à l'entrée d'un récipient de fluide.

8. Appareil suivant l'une quelconque des revendications précédentes, caractérisé en ce que le mandrin (28) comprend une mâchoire pouvant coulisser sur la canule (13) et présentant plusieurs doigts (29) s'étendant dans la direction distale, afin de s'engager dans l'évidement de la collerette (26).

9. Appareil suivant la revendication 8, caractérisé en ce que l'évidement (30) va en se rétrécissant radialement vers l'intérieur dans la direction distale.

10. Appareil suivant l'une quelconque des revendications précédentes, caractérisé en ce que la collerette (26) comporte une face distale adhésive.

**Ansprüche**

1. Apparatur zur Drainage der Pleurahöhle, mit einem Gehäuse (12) das eine innere Kammer (15) definiert, welche in Fließverbindung mit einer von dem Gehäuse ausgehenden Kanüle (13) steht, einer von dem Gehäuse ausgehenden Auslaßöffnung (14) und einer axial zur Kanüle angeordneten, mit einem elastischen Verschlußstopfen (17) verschlossenen Einlaßöffnung (16), einer abnehmbaren Nadel (23), die durch den Stopfen in die Kanüle reicht, und einem axial zur Kanüle (13) bewegbaren Flansch (26) mit einer distalen Flache, **dadurch gekennzeichnet, daß** die Kanüle (13) und das Gehäuse (12) einstückig ausgebildet sind, die innere Kammer (15) des Gehäuses in ständiger Fließverbindung mit der Auslaßöffnung (14) steht, und die Apparatur einen Ring (28) aufweist, der in eine Ausnehmung (30) in der proximalen Fläche des Flansches eingreift, um den Flansch (26) an einer axialen Bewegung zu hindern.

2. Apparatur nach Anspruch 1, dadurch gekennzeichnet, daß eine Kappe (18) für die Einlaßöffnung (16) vorgesehen ist, wobei die Kappe (18) an das Gehäuse (12) angeschlossen ist und eine Öffnung axial zur Kanüle (13) zur Durchführung der Nadel (23) aufweist.

3. Apparatur nach Anspruch 1 oder Anspruch 2, dadurch gekennzeichnet, daß der Stopfen (17) einen peripheren Flansch (20) aufweist, um die Einführung des Stopfens (17) in die Einlaßöffnung (16) zu begrenzen.

4. Apparatur nach Anspruch 3, dadurch gekennzeichnet, daß die Auslaßöffnung (14) zwischen Kanüle (13) und Einlaßöffnung (16) liegt und die distale Fläche des Stopfens (17) der Auslaßöffnung (14) gegenüberliegt.

5. Apparatur nach einem der vorhergehenden Ansprüche, dadurch gekennzeichnet, daß sie außerdem einen Drainageschlauch (31) aufweist, der an einem Ende an die Auslaßöffnung (14) dicht angeschlossen ist, wobei zwei Einwegventile (34) ausschließlich für den Ausfluß in dem Drainageschlauch (31) in Reihe angeordnet sind und wobei der Schlauch (31) zwischen den Ventilen eine Pumpenöffnung (35) als Zugang zum Innenraum des Schlauches aufweist.

6. Apparatur nach Anspruch 5, dadurch gekennzeichnet, daß sie außerdem eine Spritze (37) aufweist, welche an der Pumpenöffnung (35) befestigt ist, so daß der Innenraum der Spritze mit dem Innenraum des Schlauches (31) in Fließverbindung steht.

7. Apparatur nach Anspruch 5 oder Anspruch 6, dadurch gekennzeichnet, daß das andere Ende des Schlauches (31) mit dem Zulauf eines Flüssigkeitsbehälters dicht verbunden ist.

8. Apparatur nach einem der vorhergehenden Ansprüche, dadurch gekennzeichnet, daß der Ring (28) eine auf der Kanüle (13) verschiebbare Nabe umfaßt, und eine Vielzahl distal angeordneter Finger (29) zum Eingriff in die Ausnehmung des Flansches (26) aufweist.

9. Apparatur nach Anspruch 8, dadurch gekennzeichnet, daß sich die Ausnehmung (30) in distaler Richtung radial nach innen verjüngt.

10. Apparatur nach einem der vorhergehenden Ansprüche, dadurch gekennzeichnet, daß der Flansch (26) eine distale Klebefläche aufweist.

EP 0 232 600 B1

FIG.1

9

FIG.2

EP 0 232 600 B1